# EUROPEAN PATENT APPLICATION

(11) **EP 4 272 699 A1**
(43) Date of publication of application: **08.11.2023**
(21) Application number: 21913504.3
(22) Date of filing: 11.11.2021
(51) Int. Cl.: A61F 2/07

(54) **LUMINAL STENT**

(30) Priority: 30.12.2020 CN 202011631153
(71) Applicant: Lifetech Scientific (Shenzhen) Co., Ltd., Shenzhen, Guangdong 518063 (CN)
(72) Inventor: TANG, Chunwei, Shenzhen, Guangdong 518000 (CN)
(74) Representative: dompatent von Kreisler Selting Werner - Partnerschaft von Patent- und Rechtsanwälten mbB
(86) International application number: PCT/CN2021/130046
(87) International publication number: WO 2022/142761

(57) **Abstract**

A luminal stent (100) is provided, including a tubular main body (11) and a semi-releasing device (20) connected to the tubular main body (11). The tubular main body (11) includes a binding area (112) and a non-binding area (111) distributed in a circumferential direction. The semi-releasing device (20) includes a limiting rod (21) located outside the tubular main body (11), and binding lines (22) and limiting fasteners (23) arranged on the tubular main body (11). The binding lines (22) are arranged in the binding area (112). The binding lines (22) each include a fixed end (22a), a binding portion (22b) and a locking portion (22c) which are connected to one another. The fixed ends (22a) are fixedly connected to the tubular main body (11). Both the locking portions (22c) and the limiting fasteners (23) are detachably connected to the limiting rod (21). When both the locking portions (22c) and the limiting fasteners (23) are connected to the limiting rod (21), the binding lines (22) circumferentially constrain the binding area (112). By uniformly compressing a part of a circumferential area of the luminal stent (100), the luminal stent (100) can be in a semi-released state, thereby adjusting and positioning the luminal stent (100), and improving the accuracy of the axial and circumferential positioning of the luminal stent (100).

## Description

### Cross Reference to Related Applications

This application claims priority to Chinese Patent Application No. 202011631153.8, filed on December 30, 2020 in China National Intellectual Property Administration and entitled "Luminal Stent", which is hereby incorporated by reference in its entirety.

### Technical Field

The present application relates to the technical field of interventional medical instruments, and in particular, to a luminal stent.

### Background Art

In the past ten years, aortic endovascular covered stent exclusion has been widely used in aneurysms, arterial dissections and other lesions of thoracic and abdominal aortas, has a definite curative effect, small wound, quick recovery and fewer complications, and has become a first-line therapy. During the operation, under fluoroscopic monitoring of X-ray, a covered stent is delivered to a lesion location through a corresponding delivery system. The covered stent isolates a blood flow from the lesion location and eliminates the influence of the blood pressure on the lesion location to achieve a healing purpose.

In order to solve the positioning problem of the covered stent in vivo, developing marks are usually made at key positions of the stent, and the covered stent is axially and circumferentially positioned using the developing marks. However, when the covered stent is compressed in a delivery sheath tube, the covered stent has compression folds in its circumferential direction and is in an extended state in an axial direction. If the stent is positioned by the developing marks at this time, there will be large circumferential and axial deviations.

### Summary of the Invention

According to various embodiments of the present application, a luminal stent is provided.

A luminal stent is provided, including a tubular main body and a semi-releasing device connected to the tubular main body. The tubular main body includes a binding area and a non-binding area distributed in a circumferential direction. The semi-releasing device includes a limiting rod located outside the tubular main body, and binding lines and limiting fasteners arranged on the tubular main body. The binding lines are arranged in the binding area, and the binding lines each include a fixed end, a binding portion and a locking portion which are connected to one another. The fixed ends are fixedly connected to the tubular main body, and both the locking portions and the limiting fasteners are detachably connected to the limiting rod. When both the locking portions and the limiting fasteners are connected to the limiting rod, the binding lines circumferentially constrain the binding area.

In the luminal stent of the present application, the semi-releasing device further includes a limiting buckle; the limiting buckle is arranged on an outer surface of the tubular main body; and the binding lines are threaded through the limiting buckle.

In the luminal stent of the present application, the tubular main body includes a plurality of circles of waveform rings including a plurality of wave crests, a plurality of wave troughs, and a plurality of connecting rods respectively connected to the adjacent wave crests and wave troughs.

There are a plurality of limiting buckles; in a natural state, a distance between two adjacent limiting buckles along the circumferential direction of the tubular main body is m; a vertical distance between a fixed point of the limiting buckle and the wave trough located below the limiting buckle and closest to the limiting buckle is n; and m and n satisfy m≤2n.

In the luminal stent of the present application, the semi-releasing device includes at least one group of paired binding lines; and in the circumferential direction of the tubular main body, the fixed ends of the paired binding lines are respectively located on two sides of the limiting fasteners.

In the luminal stent of the present application, a ratio of lengths of the paired binding lines is T1, and a ratio of distances between the fixed ends of the paired binding lines and central axes of the limiting fasteners is T2, T1=T2.

In the luminal stent of the present application, the paired binding lines have equal lengths; and in the circumferential direction of the tubular main body, the distances between the fixed ends of the paired binding lines and the limiting fasteners are equal.

In the luminal stent of the present application, when the paired binding lines circumferentially constrain the binding area under the circumferential limitation of the limiting rod, the locking portions of the paired binding lines are axially limited to each other.

In the luminal stent of the present application, each locking portion is a circular or semicircular structure formed by coiling the binding line; when the paired binding lines circumferentially constrain the binding area under the circumferential limitation of the limiting rod, the locking portions of the paired binding lines are not hooked to each other, so that when the limiting rod is separated from the limiting fasteners, the paired binding lines are separated from each other.

In the luminal stent of the present application, in the paired binding lines, the binding line sleeving the limiting rod has two traction sections; and at least one of the two traction sections is threaded through the locking portion of the other binding line.

In the luminal stent of the present application, a proximal end of the limiting rod is provided with a guide head; and a ratio of a size of the guide head in a radial direction of the limiting rod to a diameter of the limiting rod is 1-1.5.

In the luminal stent of the present application, a ratio of a diameter of a circumcircle of a cross section when the luminal stent is in a semi-released state to a diameter of a circumcircle of a cross section when the luminal stent expands is 0.6-0.8.

In the luminal stent of the present application, an angle of circumference covered by the non-binding area is 180°-340°, and an angle of circumference covered by the binding area is 20°-180°.

In the luminal stent of the present application, a branch opening is formed in the non-binding area, and an edge of the branch opening is provided with a developing structure.

In the luminal stent of the present application, a size of the limiting buckle is 100%-200% of a cross-sectional area of the binding line.

In the luminal stent of the present application, the limiting buckle is located in the middle of the connecting rod of the waveform ring.

In summary, implementing the luminal stent of the present application has the following beneficial effects. According to the present application, a luminal stent is provided with a semi-releasing device including a limiting rod located outside a tubular main body, and binding lines and limiting fasteners arranged on the tubular main body, and the tubular main body includes a binding area and a non-binding area distributed in a circumferential direction; the binding lines are arranged in the binding area; the binding lines each include a fixed end, a binding portion and a locking portion which are connected to one another; the fixed ends are fixedly connected to the tubular main body; both the locking portions and the limiting fasteners are detachably connected to the limiting rod; and when both the locking portions and the limiting fasteners are connected to the limiting rod, the binding lines circumferentially constrain the binding area. By uniformly compressing a part of a circumferential area of the luminal stent, the luminal stent can be in a semi-released state, thereby adjusting and positioning the luminal stent, and improving the accuracy of the axial and circumferential positioning of the luminal stent.

### Brief Description of the Drawings

The present application is further described below with reference to the accompanying drawings and embodiments. Among the drawings:
Fig. 1 is a schematic diagram when a luminal stent provided by Embodiment I of the present application is in a semi-released state;
Fig. 2 is an enlarged diagram of portion A of the luminal stent shown in Fig. 1;
Fig. 3 is a schematic diagram when the luminal stent shown in Fig. 1 completely expands;
Fig. 4 is a schematic diagram when a luminal stent of one embodiment is in a semi-released state;
Fig. 5 is a schematic diagram when the luminal stent shown in Fig. 1 completely expands;
Fig. 6 is a schematic diagram when a binding line of a luminal stent of one embodiment crosses a wave trough;
Fig. 7 is a schematic diagram of a limiting buckle of a luminal stent of one embodiment;
Fig. 8 is a schematic diagram when a binding line of a luminal stent of one embodiment does not cross a wave trough;
Fig. 9 is a schematic diagram when a luminal stent provided by Embodiment II of the present application is in a semi-released state;
Fig. 10 is an enlarged diagram of portion B of the luminal stent shown in Fig. 8;
Fig. 11 is a schematic diagram when the luminal stent shown in Fig. 9 completely expands;
Fig. 12 is a schematic diagram when a luminal stent provided by Embodiment III of the present application is in a semi-released state;
Fig. 13 is an enlarged diagram of portion C of the luminal stent shown in Fig. 11;
Fig. 14 is a schematic diagram when the luminal stent shown in Fig. 11 completely expands;
Fig. 15 is a schematic diagram when locking portions of paired binding lines of a luminal stent of one embodiment are located at different axial positions of a limiting rod;
Fig. 16 is a schematic diagram illustrating that the paired binding lines shown in Fig. 14 pull the limiting rod to be bent;
Fig. 17 is a schematic connection diagram of locking portions of paired binding lines in a luminal stent of one implementation relative to a limiting rod; and
Fig. 18 is a schematic connection diagram of locking portions of paired binding lines in a luminal stent of another implementation relative to a limiting rod.

### Detailed Description of the Invention

In order to make the foregoing objectives, features and advantages of the present application more obvious and understandable, the specific implementation modes of the present application are described in detail with reference to the accompanying drawings. Many specific details are described in the following descriptions to facilitate full understanding of the present application. However, the present application can be implemented in a variety of other ways than those described herein, and those skilled in the art can make similar improvements without departing from the connotation of the present application. Therefore, the present application is not limited by specific implementations disclosed below.

It should be noted that when an element is referred to as being "fixed" or "arranged" to another element, it can be directly on another element or an intermediate element may also exist. When one element is considered to be "connected" to another element, it can be directly connected to another element or an intermediate element may also exist at the same time. The terms "perpendicular", "horizontal", "left", "right" and similar expressions used herein are for illustrative purposes only, and are not meant to be the only implementation modes.

Unless otherwise defined, all technical and scientific terms used herein have the same meanings as those commonly understood by those skilled in the art of the present application. The terms used in the specification of the present application herein are only for the purpose of describing specific implementations, and are not intended to limit the present application. The term "and/or" used herein includes any and all combinations of one or more related listed items.

It should be noted that it is defined that an end of a luminal stent close to the heart after the stent is released is a proximal end, and an end far away from the heart is a distal end.

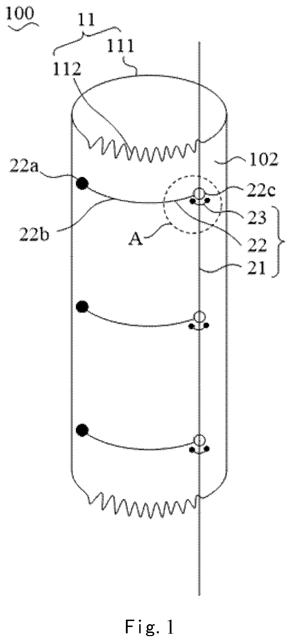

Referring to Fig. 1, a luminal stent 100 provided by an embodiment of the present application includes a tubular main body 11, and a semi-releasing device 20 connected to the tubular main body 11 and circumferentially constraining the tubular main body 11. The semi-releasing device 20 includes a limiting rod 21, binding lines 22 and limiting fasteners 23. The limiting rod 21 is located outside the tubular main body 11, and the binding lines 22 and the limiting fasteners 23 are both arranged on the tubular main body 11.

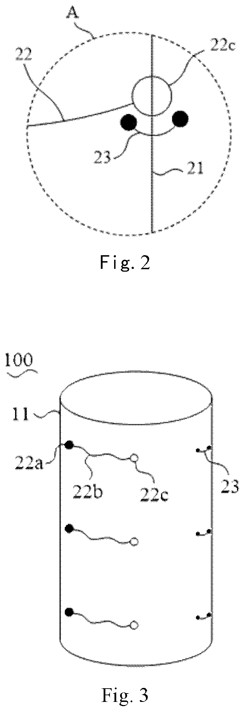

The tubular main body 11 includes a binding area 112 and a non-binding area 111 distributed in a circumferential direction. The binding lines 22 are arranged in the binding area 112. The binding lines 22 each include a fixed end 22a, a binding portion 22b and a locking portion 22c. The fixed ends 22a are fixedly connected to the tubular main body 11. As shown in Fig. 2, both the locking portions 22c and the limiting fasteners 23 are detachably connected to the limiting rod 21. When both the locking portions 22c and the limiting fasteners 23 are connected to the limiting rod 21, the binding lines 22 circumferentially constrain the binding area 112. Referring to Fig. 3, when the limiting rod 21 is separated from the locking portions 22c and the limiting fasteners 23, the limiting rod 21 releases the binding of the locking portions 22c, and then the binding lines 22 release the circumferential constraint on the binding area 112, so that the tubular main body 11 completely expands.

Since the binding lines 22 are fixed on the tubular main body 11 by the fixed ends 22a, when the locking portions 22c are released by the limiting rod 21, the fixed ends 22a can prevent the binding lines 22 from being separated from the luminal stent 100 and entering a downstream blood vessel.

In the present application, a semi-releasing device 20 is arranged on the luminal stent 100. When the luminal stent 100 is released from a delivery sheath tube, the luminal stent 100 is in a semi-released state under the constraint of the semi-releasing device 20. At this time, the luminal stent 100 is not adhered to the vascular wall, and an operator can still adjust the axial and circumferential positions of the luminal stent 100. After accurate positioning is achieved, the constraint from the semi-releasing device 20 is released to make the luminal stent 100 expand and be adhered to the wall.

It can be understood that if a diameter of a circumcircle of a cross section of the luminal stent 100 is too large when the luminal stent is in the semi-released state, the stent is easily adhered to the wall, which is not conducive to the axial and circumferential adjustment. If the diameter of the circumcircle of the cross section of the luminal stent 100 is too small when the luminal stent is in the semi-released state, the semi-releasing effect is not significant, and there is still a large circumferential and axial positioning deviation. Thus, in this embodiment, a ratio of a diameter of a circumcircle of a cross section when the luminal stent 100 is in the semi-released state to a diameter of a circumcircle of a cross section when the luminal stent 100 expands is about 0.6-0.8.

An angle of circumference covered by the non-binding area 111 is about 180°-340°, that is, an angle of circumference covered by the binding area 112 is about 20°-180°, so that the circumferential constraint of the binding lines 22 on the binding area 112 would be controlled within a reasonable range, which is conductive to the axial and circumferential adjustment of the luminal stent 100 in the semi-released state.

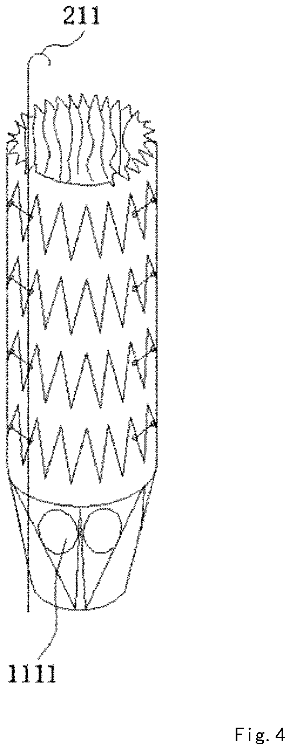

As shown in Fig. 4, a branch opening 1111 is formed in the non-binding area 111, and an edge of the branch opening 1111 is provided with a developing structure. When the semi-releasing device 20 constrains the binding area 112, the branch opening 1111 in the non-binding area 111 is in a complete expansion state. When the branch opening 1111 is positioned, the branch opening 1111 in the expansion state and the developing structure are positioned more accurately. The semi-releasing device 20 constrains the binding area 112 only, which is particularly conductive to positioning of a luminal stent with a branch opening 1111.

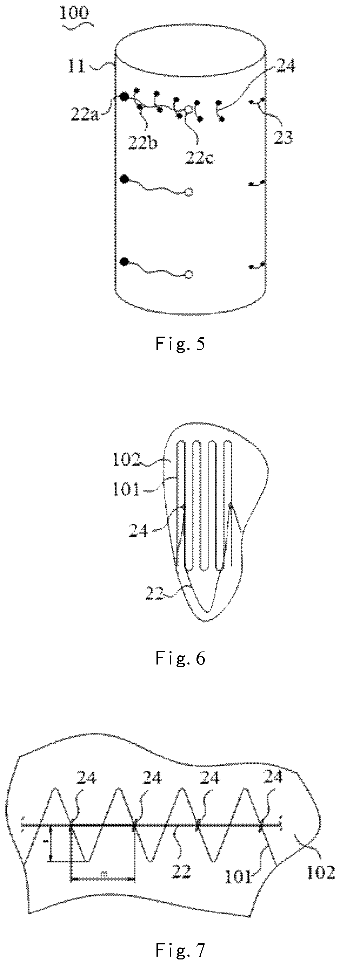

Referring to Fig. 5, the semi-releasing device 20 also includes a limiting buckle 24. The limiting buckle 24 is arranged on an outer surface of the tubular main body 11, and the binding lines 22 are threaded through the limiting buckle 24, so that the binding lines 22 can uniformly compress the tubular main body 11 to improve the accuracy of overall positioning of the stent. Moreover, when the luminal stent 100 is compressed in the delivery sheath tube or the constraint of the binding lines 22 on the luminal stent 100 is released, the limiting buckle 24 can also prevent the binding lines 22 from axially moving. Of course, in other embodiments, the limiting buckle 24 can also be arranged on an inner surface of the tubular main body 11.

It should be noted that there are many possibilities for the luminal stent 100 to form the structure of the tubular main body 11, as long as the luminal stent 100 forms a hollow luminal structure, so that the lumen of the luminal stent 100 is formed into a channel for blood circulation.

In some implementations, the tubular main body 11 includes a plurality of circles of waveform rings 101 (as shown in Fig. 7), each waveform ring 101 includes a plurality of wave crests, a plurality of wave troughs, and a plurality of connecting rods respectively connected to adjacent wave crests and wave troughs. The plurality of circles of waveform rings 101 are arranged in turn from the proximal end to the distal end. In some embodiments, the waveform rings are arranged in parallel at intervals. Each waveform ring 101 is of a closed cylindrical structure. The plurality of circles of waveform rings 101 can have the same or similar waveform shapes. It can be understood that this embodiment does not define the specific structure of the waveform ring 101. The waveform of the waveform ring 101 can be set as required, and the number of waveforms in each circle of waveform ring 101 and a waveform height can be set as required.

The plurality of circles of waveform rings 101 are connected with a covering film 102. The plurality of circles of waveform rings 101 are made of a highly biocompatible material, such as nickel-titanium and stainless steel. The covering film 102 is made of a highly biocompatible polymer material, such as polytetrafluoroethylene (PTFE), fluorinated ethylene propylene copolymer (FEP), and polyethylene terephthalate (PET).

The size of the limiting buckle 24 is about 100%-200% of a cross-sectional area of the binding line 22. If the size of the limiting buckle 24 is too large, the axial movement range of the binding line 22 is large, which affects the radial compression effect of the stent. If the size of the limiting buckle 24 is too small, the friction between the limiting buckle 24 and the binding line 22 will increase, which will affect the relative movement between the binding line 22 and the limiting buckle 24 along the circumferential direction of the tubular main body 11, and is not conducive to smooth expansion of the luminal stent 100.

There may be a plurality of limiting buckles 24. The multiple limiting buckles 24 are uniformly distributed at the binding area 112 along the circumferential direction of the tubular main body 11, so that the binding lines 22 are hung on the outer surface of the tubular main body 11 by using the plurality of limiting buckles 24 to well position the binding lines 22, and the stability and accuracy of the circumferential constraint of the binding lines 22 on the binding area 112 are improved.

Referring to Fig. 6, if two limiting buckles 24 are too far away from each other, when the luminal stent 100 is in a fully radially compressed state in the delivery sheath tube, the binding lines 22 are in a loose state, and the binding line 22 between the two limiting buckles 24 will move axially, and even cross the wave trough of the waveform ring 101 and be hooked on the wave trough. When the luminal stent 100 is completely released from the delivery sheath tube, because the binding line 22 is hooked on the waveform ring 101, the luminal stent 100 cannot expand normally.

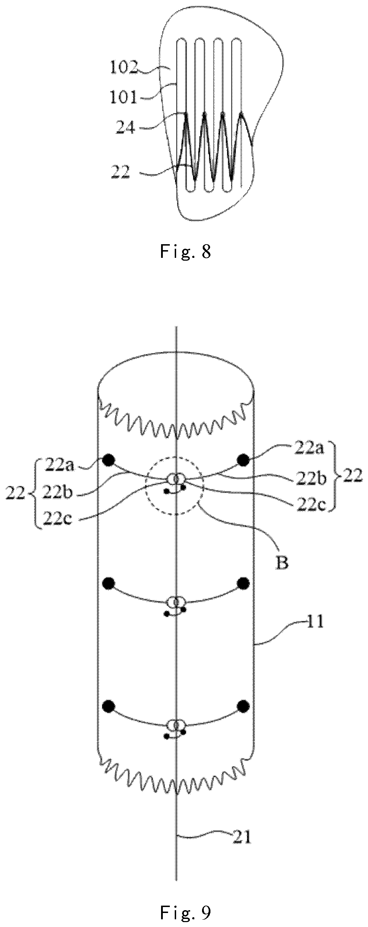

Referring to Fig. 7, in the natural state (i.e. when the luminal stent 100 is completely released), a distance between two adjacent limiting buckles 24 along the circumferential direction of the tubular main body 11 is m, that is, an arc length of a partial structure, located between the two adjacent limiting buckleA vertical distance between a fixed point of the limiting buckand m and n satisfy m≤2n. Referring to Fig. 8, when the luminal stent 100 is in the fully radially compressed state in the delivery sheath tube, the binding line 22 of the luminal stent 100 in the radially compressed state can be prevented from crossing the wave trough of the waveform ring 101.

In this embodiment, as shown in Fig. 1, there are a plurality of binding lines 22, which are distributed axially at intervals. The binding line 22 may be a flexible line with high tensile strength, such as a PTFE line and a polyester suture. The fixed ends 22a can be fixed on the covering film 102 or the plurality of circles of waveform rings 101 by knotting or the like. The binding portions 22b of the binding lines 22 may be composed of a single flexible line or a plurality of flexible lines. The locking portion 22c may be selected from, but is not limited to, a binding line ring, a metal ring and the like. The limiting buckles 24 are all arranged on the waveform rings 101, which is not only conducive to guiding the binding lines 22 threaded through the limiting buckles 24 to radially compress the stent, but also can lower the risk of damage to the covering film by the binding lines 22. In some embodiments, the limiting buckles 24 are located in the middle of the connecting rods of the waveform rings 101, so as to apply a uniform force to the waveform rings 101.

The limiting rod 21 may be selected from a metal guide wire with good elastic memory and low surface roughness, such as a nickel titanium wire that has the physical property meeting the requirements and has good biocompatibility with the human body. A diameter of the limiting rod 21 may be about 0.2-0.6 mm, so that the diameter of the limiting rod 21 is appropriate. Increase of the overall profile (i.e., a passing area) of the luminal stent 100 due to an extremely large diameter of the limiting rod 21 is avoided. If the diameter of the limiting rod 21 is too large, the supporting force is low, which affects the constraint on the locking portions 22c and the limiting fasteners 23.

In some implementations, the surface roughness of the limiting rod 21 is less than or equal to about 0.2 µm, so that the limiting rod 21 can be smoothly withdrawn out from the locking portions 22c and the limiting fasteners 23 to release the constraint on the locking portions 22c and the limiting fasteners 23.

In some implementations, as shown in Fig. 4, the proximal end of the limiting rod 21 is provided with a guide head 211. The guide head 211 is used for guiding the limiting rod 21 to pass through the limiting fasteners 23, and the guide head 211 is used to reduce the damage to a blood vessel caused by the limiting rod 21.

In some implementations, the guide head 211 is a soft head arranged at the proximal end of the limiting rod 21. Therefore, the soft head is used to avoid the damage to the blood vessel caused by the proximal end of the limiting rod 21. Specifically, a length of the soft head is set to be about 10-20 mm. This setting can not only maintain the constraint of the limiting rod 21 to the locking portions 22c and the limiting fasteners 23, but also prevent the limiting rod 21 from damaging the blood vessel. If the soft head is too long, part of the soft head is easily at a constraining position of the limiting rod 21 to the binding lines 22. In this way, the locking portions 22c or the limiting fasteners 23 may be loosened from the limiting rod 21 due to the low supporting strength of the soft head. If the soft head is too short, the soft head is not flexible enough, which will easily damage the blood vessel.

In some other implementations, the guide head 211 is a ball head formed at the proximal end of the limiting rod 21. For example, when the limiting rod 21 is made of a metal guide wire, the ball head can be formed at the proximal end of the limiting rod 21 by spot welding.

A diameter of the ball head may be about 100%-150% of the diameter of the limiting rod 21. A guide wire with poor elastic memory is easy to bend after being stressed, which will increase the resistance when the guide wire is pulled out. The diameter of the ball head is about 0.3-0.6 mm.

The diameter of the guide wire should not be too large or too small. If the diameter is too large, the overall profile of the stent will increase. If the diameter is too small, the supporting force will be not enough to constrain two ends of a long thread buckle. If the diameter of the ball head at the proximal end of the guide wire is less than 0.3 mm, the blood vessel will be easily punctured. If the diameter is greater than 150% of the diameter of the guide wire, a position close to the ball head and the long thread buckle will be fastened when the guide wire is pulled out, resulting in a failure of loosening.

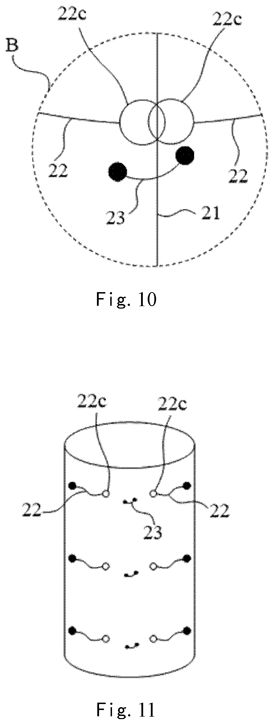

Referring to Fig. 9, Fig. 10 and Fig. 11, Embodiment II of the present application provides a luminal stent 100, which is generally the same as the luminal stent 100 of Embodiment I. The luminal stent 100 includes a tubular main body 11, and a semi-releasing device 20 connected to an outer surface of the tubular main body 11. The semi-releasing device 20 includes a limiting rod 21 located outside the tubular main body 11, and binding lines 22 wound outside the tubular main body 11.

A difference between Embodiment II and Embodiment I is that the semi-releasing device 20 includes at least one group of paired binding lines 22. In the circumferential direction of the tubular main body 11, the fixed ends 22a of the paired binding lines 22 are respectively located on two sides of the limiting fasteners 23.

In this embodiment, by means of the paired binding lines 22, when the limiting rod 21 is separated from the locking portions 22c of the paired binding lines 22, the paired binding lines 22 can simultaneously release the constraint on the binding area 112 of the tubular main body 11, so that the luminal stent 100 can expand and be adhered to the wall more quickly. At the same time, since the fixed ends 22a of the paired binding lines 22 are respectively located on two sides of the limiting fasteners 23, when the paired binding lines 22 release the constraint on the binding area 112 of the tubular main body 11, the binding area 112 expands towards the fixed ends 22a located on two sides of the limiting fasteners 23, that is, the binding area 112 expands towards two opposite directions along the circumferential direction of the luminal stent 100, so as to offset a circumferential force generated by the luminal stent 100 during the expansion. In this way, the releasing stability of the luminal stent 100 is improved, and the positioning is more accurate.

A ratio of lengths of the paired binding lines 22 is T 1, and a ratio of distances between the fixed ends 22a of the paired binding lines 22 and central axes of the limiting fasteners 23 is T2, T1=T2. The central axis of each limiting fastener 23 can be defined by the limiting rod 21 threaded through the limiting fastener 23. Specifically, a longitudinal axis of the limiting rod 21 overlaps the central axis of the limiting fastener 23 regardless of a shape and position error of the longitudinal axis of the limiting rod 21 relative to the tubular main body 11 caused by the movement of the limiting rod 21 in the limiting fastener 23. In this embodiment, the ratio of the lengths of the paired binding lines 22 is set to be equal to the ratio of the distances from the fixed ends 22a of the binding lines to the central axes of the limiting fasteners 23, so that circumferential force of the binding area 112 that expands in two opposite directions can be well offset while the paired binding lines 22 simultaneously release the circumferential constraint on the binding area 112.

For example, in some implementations, the lengths of the paired binding lines 22 are equal. In the circumferential direction of the tubular main body 11, the distances between the fixed ends 22a of the paired binding lines 22 and the limiting fasteners 23 are equal. By means of this structural setting, the paired binding lines 22 circumferentially constrain the binding area 112 to the same extent. Therefore, when the limiting rod 21 is separated from the locking portions 22c of the paired binding lines 22, the paired binding lines 22 release the circumferential constraint on the binding area 112 at the same time. The circumferential forces of the binding area 112 that expands in two opposite directions are basically the same, which can be well offset, making the luminal stent 100 released more stably and positioned more accurately.

It can be understood that the present application does not limit the number of groups of paired binding lines 22. For example, as shown in Fig. 8, the semi-releasing device 20 includes three groups of paired binding lines 22. In other implementations, the semi-releasing device 20 may also be provided with two or more groups of binding lines 22. Alternatively, the semi-releasing device 20 may also be provided with non-paired binding lines 22 in addition to the paired binding lines 22, so that some of the binding lines 22 are arranged in pairs, and the other part of the binding lines 22 are not arranged in pairs, thus improving the flexibility of the axial constraining process of the binding area 112.

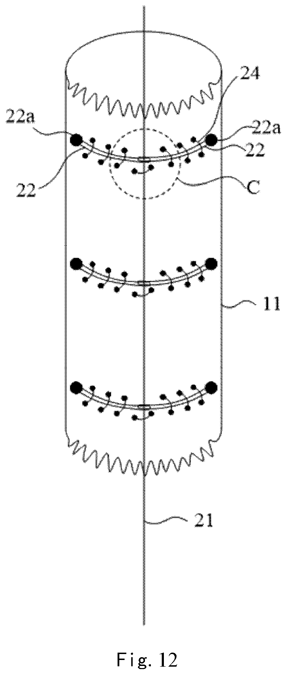

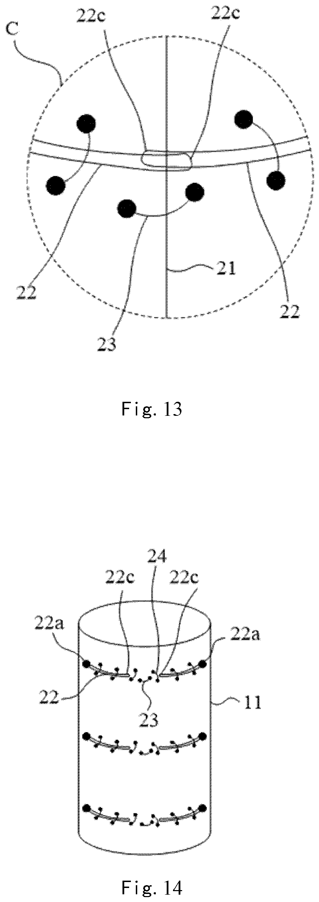

Referring to Fig. 12, Fig. 13 and Fig. 14, Embodiment III of the present application provides a luminal stent 100, which is generally the same as the luminal stent 100 of Embodiment I. The luminal stent 100 includes a tubular main body 11, and a semi-releasing device 20 connected to an outer surface of the tubular main body 11.

A difference between Embodiment III and Embodiment I is that when the paired binding lines 22 circumferentially constrain the binding area 112 under the circumferential limitation of the limiting rod 21, the locking portions 22c of the paired binding lines 22 are axially limited to each other to ensure that positions where the locking portions 22c of the paired binding lines 22 apply an acting force on the limiting rod 21 are consistent, so as to avoid the risk that the limiting rod 21 bends and skews since the acting forces applied by the paired binding lines 22 on the limiting rod 21 are not at the same position in the axial direction of the limiting rod 21. By means of this structural setting, when the limiting rod 21 constrains the paired binding lines 22, the paired binding lines 22 can stably maintain the binding area 112 in a compressed state, thereby effectively improving the overall stability of the luminal stent 100.

Each locking portion 22c is of a circular or semi-circular structure formed by coiling the binding line 22. When the paired binding lines 22 circumferentially constrain the binding area 112 under the circumferential limitation of the limiting rod 21, the locking portions 22c of the paired binding lines 22 are not hooked to each other, so that when the limiting rod 21 is separated from the limiting fasteners 23, the paired binding lines 22 are separated from each other to meet the requirement of releasing the circumferential constraint on the binding area 112.

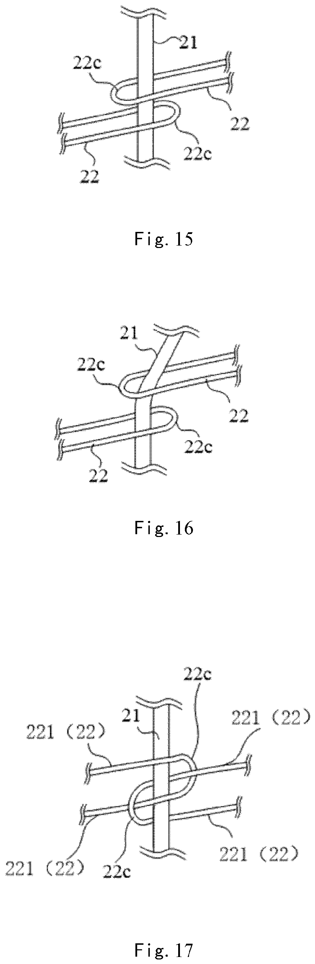

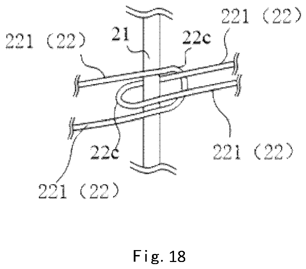

As shown in Fig. 17 and Fig. 18, in the paired binding lines 22, the binding line 22 sleeving the limiting rod 21 has two traction sections 221, and at least one of the two traction sections 221 is threaded through the locking portion 22c of the other binding line 22.

It should be noted that when the locking portion 22c is of a circular structure, only one locking portion 22c of the paired binding lines 22 sleeves the limiting rod 21 and is threaded through the locking portion 22c of the other binding line 22. In this way, the paired binding lines 22 are all bound to the limiting rod 21, and are limited to each other along the axial direction of the limiting rod 21. That is, under this structural setting, the locking portions 22c of the paired binding lines 22 are bound to a unified position, and will not axially move relative to each other, which can avoid the risk that the limiting rod 21 bends and skews since the acting forces applied by the paired binding lines 22 on the limiting rod 21 are not at the same position in the axial direction of the limiting rod 21.

For example, as shown in Fig. 15 and Fig. 16, if two binding lines 22 are not constrained to each other along the axial direction of the limiting rod 21, the two binding lines 22 apply opposite acting forces on the limiting rod 21, so that the locking portions 22c of the two binding lines 22 can move relative to each other along the axial direction of the limiting rod 21 because they are not constrained to each other. In this way, the limiting rod 21 may be pulled to be bent since the acting forces applied by the paired binding lines 22 on the limiting rod 21 may be not at the same position in the axial direction of the limiting rod 21, which affects the stability of the limiting rod 21 constraining the luminal stent 100 in the semi-released state.

The technical features of the embodiments described above can be arbitrarily combined. In order to make the description concise, all possible combinations of various technical features in the above embodiments are not completely described. However, the combinations of these technical features should be considered as the scope described in the description as long as there is no contradiction in them.

The foregoing embodiments represent only a few implementation modes of the present application, and the descriptions are specific and detailed, but should not be construed as limiting the patent scope of the present application. It should be noted that those of ordinary skill in the art may further make variations and improvements without departing from the conception of the present application, and these variations and improvements all fall within the protection scope of the present application. Therefore, the patent protection scope of the present application should be subject to the appended claims.

## Claims

1. A luminal stent, comprising a tubular main body, and a semi-releasing device connected to the tubular main body, wherein the tubular main body comprises a binding area and a non-binding area distributed in a circumferential direction; the semi-releasing device comprises a limiting rod located outside the tubular main body, and binding lines and limiting fasteners arranged on the tubular main body; the binding lines are arranged in the binding area; the binding lines each comprise a fixed end, a binding portion and a locking portion which are connected to one another; the fixed ends are fixedly connected to the tubular main body; both the locking portions and the limiting fasteners are detachably connected to the limiting rod; when both the locking portions and the limiting fasteners are connected to the limiting rod, the binding lines circumferentially constrain the binding area.

2. The luminal stent according to claim 1, wherein the semi-releasing device further comprises a limiting buckle; the limiting buckle is arranged on an outer surface of the tubular main body; and the binding lines are threaded through the limiting buckle.

3. The luminal stent according to claim 2, wherein the tubular main body comprises a plurality of circles of waveform rings comprising a plurality of wave crests, a plurality of wave troughs, and a plurality of connecting rods respectively connected to the adjacent wave crests and wave troughs; and
there are a plurality of limiting buckles; in a natural state, a distance between two adjacent limiting buckles along the circumferential direction of the tubular main body is m; a vertical distance between a fixed point of the limiting buckle and the wave trough located below the limiting buckle and closest to the limiting buckle is n; and m and n satisfy m≤2n.

4. The luminal stent according to claim 1, wherein the semi-releasing device comprises at least one group of paired binding lines; and in the circumferential direction of the tubular main body, the fixed ends of the paired binding lines are respectively located on two sides of the limiting fasteners.

5. The luminal stent according to claim 4, wherein a ratio of lengths of the paired binding lines is T1, and a ratio of distances between the fixed ends of the paired binding lines and central axes of the limiting fasteners is T2, T1=T2.

6. The luminal stent according to claim 4, wherein the paired binding lines have equal lengths; and in the circumferential direction of the tubular main body, the distances between the fixed ends of the paired binding lines and the limiting fasteners are equal.

7. The luminal stent according to any one of claims 4 to 6, wherein when the paired binding lines circumferentially constrain the binding area under the circumferential limitation of the limiting rod, the locking portions of the paired binding lines are axially limited to each other.

8. The luminal stent according to claim 7, wherein each locking portion is a circular or semicircular structure formed by coiling the binding line; when the paired binding lines circumferentially constrain the binding area under the circumferential limitation of the limiting rod, the locking portions of the paired binding lines are not hooked to each other, so that when the limiting rod is separated from the limiting fasteners, the paired binding lines are separated from each other.

9. The luminal stent according to claim 7, wherein in the paired binding lines, the binding line sleeving the limiting rod has two traction sections; and at least one of the two traction sections is threaded through the locking portion of the other binding line.

10. The luminal stent according to claim 1, wherein a proximal end of the limiting rod is provided with a guide head; and a ratio of a size of the guide head in a radial direction of the limiting rod to a diameter of the limiting rod is 1-1.5.

11. The luminal stent according to claim 1, wherein a ratio of a diameter of a circumcircle of a cross section when the luminal stent is in a semi-released state to a diameter of a circumcircle of a cross section when the luminal stent expands is 0.6-0.8.

12. The luminal stent according to claim 1, wherein an angle of circumference covered by the non-binding area is 180°-340°, and an angle of circumference covered by the binding area is 20°-180°.

13. The luminal stent according to claim 1, wherein a branch opening is formed in the non-binding area, and an edge of the branch opening is provided with a developing structure.

14. The luminal stent according to claim 2, wherein a size of the limiting buckle is 100%-200% of a cross-sectional area of the binding line.

15. The luminal stent according to claim 3, wherein the limiting buckle is located in the middle of the connecting rod of the waveform ring.
